# EUROPEAN PATENT APPLICATION

(11) **EP 3 725 303 A1**
(43) Date of publication of application: **21.10.2020**
(21) Application number: 20170456.6
(22) Date of filing: 20.04.2020
(51) Int. Cl.: A61K 9/08, A61K 31/722, A61P 11/12

(54) **METHOD FOR PREPARING A PLANT-BASED FUNGAL CHITOSAN PRODUCT**

(30) Priority: 18.04.2019 IT 201900006104
(71) Applicant: S.I.I.T. S.r.l.-Servizio Internazionale Imballaggi Termosaldanti, 20090 Trezzano sul Naviglio MI (IT)
(72) Inventor: Costa, Andrea, 20090 Trezzano sul Naviglio (MI) (IT); Madaro, Elena, 20090 Trezzano sul Naviglio (MI) (IT); Dominoni, Mirko, 20090 Trezzano sul Naviglio (MI) (IT)
(74) Representative: Hoffmann Eitle

(57) **Abstract**

The present invention relates to a method for preparing a liquid composition in the form of a homogeneous solution comprising a chitosan, to the liquid composition in the form of a homogeneous solution that can be obtained by means of said method and to the use thereof in a method for the treatment of diseases and/or disorders of the respiratory airways connected to a hypersecretion of mucus.

## Description

The present invention relates to a liquid composition in the form of an aqueous homogeneous solution comprising a chitosan, preferably a plant-based fungal chitosan, as well as to a method for preparing a liquid composition in the form of a homogeneous solution comprising a chitosan, preferably a plant-based fungal chitosan. Furthermore, the present invention relates to a liquid composition in the form of a homogeneous solution that can be obtained by means of said method. Lastly, the present invention relates to a use of said composition in a method for the treatment of diseases and/or disorders of the respiratory airways related to hypersecretion of mucus.

It is known that catarrhal forms in general, and rhinitides in particular, are among the most common respiratory disorders in the human population and frequently affect subjects of adolescent and paediatric age. The most common symptoms are nasal congestion and irritation of the respiratory airways, particularly of the nose and of the throat.

Catarrh (also, and more correctly, called sputum) is the material that is secreted by the glands of the respiratory mucous membranes; catarrh consists of various elements (tracheobronchial secretions and paranasal sinuses, desquamated epithelial cells and saliva). Usually, secretions produced over a period of 24 hours can range between 25 and 100 ml; such secretions (the so-called mucus) perform important functions such as the necessary humidification of the respiratory tract and the capture of microorganisms; the physiological production of mucus is then swallowed without giving rise to any particular phenomenon.

Decongestant formulations for topical use and mucolytic formulations which act through the direct fluidification of mucus for the treatment of said respiratory airways disorders which cause a hypersecretion of mucus, in particular in the nose and in the throat, are available on the market. However, mucolytic formulations are not always effective and decongestant formulations reveal some drawbacks and adverse effects which limit their use, particularly in subjects of paediatric or adolescent age.

Therefore, it is felt by the operators of the field the need to have available new formulations/compositions which allow to obtain a rapid elimination of a secretion of excess mucus in the respiratory airways, nose and throat in particular, which arise from disorders related to illness, catarrh, common cold, flu, nasal infections, rhinitis, allergic rhinitis and sinusitis.

Mucus is a viscous colloidal secretion rich in glycoproteins, water and salts that covers the mucous membranes of the respiratory airways and it performs, among others, a lubricating and biological barrier function against infectious agents. When infectious, allergenic or irritant agents trigger an inflammatory reaction, the body reacts by increasing mucus secretion which causes congestion of the respiratory airways, nose and throat, causing difficulty in breathing. The main components of mucus are mucins (or mucin), negatively charged high molecular weight proteins. Mucins in an aqueous medium tend to aggregate acting as a mucus thickening agent. At physiological pH the mucus adhering to the mucous membrane carries with it a partial negative charge due to the presence of sialic acid and sulphated residues in the mucins. Consequently, mucins present in the mucus at contact with a macromolecule having a partial positive charge may give rise to an interaction known as mucoadhesion. Some mucolytic agents of the prior art comprise macromolecules with partial positive charge which act by means of said mucoadhesion interaction dissociating the internal bonds of the mucins and determining their precipitation. The removal of mucins from the mucus results in the fluidification of the mucus.

An example of a macromolecule with partial positive charge used as the active component of a mucolytic is chitosan, a linear polymer of beta-(1,4)-D-glucosamine. Chitosan with a partial degree of deacetylation has free amino groups. In acidic aqueous solutions, said chitosan passes into solution due to the presence of positive charges on the amino groups and it becomes mucoadhesive since it interacts with the negative charges present in the sialic acid residues of the mucins. Chitosan-mucin interactions would be high using chitosan compositions at acid pH, i.e. having high positive charge density. However, it is not possible to use compositions comprising chitosan at acid pH in the treatment of the respiratory airways, since this acidity would cause burning sensations and damage to tissues/mucous membranes of the respiratory airways (i.e. nose and throat). Thus, there arises the need to use chitosan-based compositions as mucolytics at pH compatible with the mucous membranes of the respiratory airways. At the same time, the formulation of aqueous chitosan-based compositions at pH compatible with the mucous membranes of the respiratory airways is not easy to obtain, since chitosan is a macromolecule.

EP2942063 A1 discloses chitosan-based aqueous liquid compositions at pH 6-7 in the form of emulsions for use in the treatment of disorders related to common cold by fluidising the mucus and a method for the preparation thereof. Said preparation method provides for mixing a chitosan with an emulsifying polymer (i.e. cross-linked copolymers or homopolymers) to obtain a dispersion or suspension of chitosan and emulsifying polymer in water, in other words to obtain an emulsion. The emulsions, as well as the dispersions and suspensions, if not adequately stirred mechanically before their administration, suffer from phenomena of stratification and inhomogeneity of the components contained therein which alter the repeatability of the administered dose.

As known, the emulsions per se are subject to phase separation making the compositions in the form of emulsions not stable in the long term, in particular if they are not stored under appropriate environmental conditions (e.g. temperature, humidity). Consequently, the compositions in the emulsion form have both a limited shelf life and a limited use time from the opening date of the package containing the composition. Furthermore, the intrinsic instability of the compositions formulated in the form of emulsions makes the effectiveness of the compositions uncertain.

Document US 5 422 116 A discloses a sustained release system in solution for ophthalmic use comprising chitosan with an 80% degree of deacetylation and sodium chloride in a hydrochloric acid aqueous solution. Example 2 and Example 8 show chitosan solutions with pH values of 4.85 and 5.0, respectively. In such document, chitosan is used according to its pH-dependent release rate, so as to allow a controlled release of active ingredients for ophthalmic use.

Document JP 2003 171469 A discloses an antimicrobial coating agent obtained by means of a preparation method in which a chitosan aqueous solution prepared by neutralising acid chitosan using carbonate or hydrogen carbonate ions. The method of such document can be reproduced on a large scale, at low cost and it allows to obtain a reduction of bacterial load, for example on packages for food use.

Document US 2018/147320 A1 discloses a homogeneous, aqueous and injectable chitosan solution comprising from 0.1% to 4.5% by weight of chitosan with a degree of acetylation lower than 20%, wherein said solution has a pH value higher than 6.2. Said aqueous solution is homogeneous at room temperature and is neutralised (for example see Example 2) to a pH value of 6.8 by means of a β-glycerophosphate sodium salt, an anionic surfactant, which is dispersed in an aqueous solution manually.

Document WO 2015/082965 A2 discloses a nasal rinse composition comprising sodium chloride, sodium bicarbonate, and chitosan. Such composition is produced in solid form, and solubilised in water at the time of use, thus prolonging a preservation time of the solid form without using preservatives.

The technical problem addressed and solved by the present invention lies in providing a method for preparing chitosan-based compositions in a stable liquid form and not subject to phase separation, such compositions being suitable for the effective treatment of disorders related to illness, catarrh, common cold, flu, nasal infections, rhinitis, allergic rhinitis and sinusitis. Specifically, the technical problem addressed and solved by the present invention lies in providing chitosan-based compositions in a stable liquid form and not subject to phase separation (clear solutions without precipitate) which also have a low and stable microbial load over time without using gamma rays (the compositions must not be subjected to gamma rays) and which are also easy and pleasant to administer without causing discomfort (throat irritation), for example in the nose or throat, of the users who may be, for example, children, adolescents, adults and the elderly.

In order to overcome said technical problems, after a long and intense activity of research and experimentation the Applicant developed an innovative method (in short, method of the invention) for preparing chitosan-based liquid compositions in the form of homogeneous solutions in the absence of emulsifiers (in short, compositions of the invention). The method developed by the Applicant is capable of preparing liquid compositions, in the form of homogeneous aqueous solutions, comprising plant-based fungal chitosans which are stable, clear, with a low and stable microbial load (bacteria, moulds and yeasts each individually lower than 10 CFU/g) over time and non-irritating at application site (nose and throat).

Being in the form of homogeneous solutions (i.e. solutions stable in a single phase), the compositions of the invention are not subject to phase separation and, therefore, have a longer shelf life and less demanding storage conditions, at room temperature, with respect to chitosan-based compositions in the form of emulsions of the prior art. The compositions of the present invention are stable over time as they remain clear without giving rise to a phase separation (clear solutions without precipitate) allowing constant puff administration of an active ingredient over time.

Furthermore, the method of the invention is easy to implement, economically advantageous and repeatable, since the absence of the use of emulsifiers allows to reduce the number of steps in the production method, and to reduce the number of raw materials required for the production of the chitosan-based liquid composition. The compositions of the present invention do not need to be produced using a method which provides for a sterilisation step by means of gamma rays. Thanks to the formulation developed by the Applicant, the compositions of the present invention have a low and stable microbial load (bacteria, moulds and yeasts each individually lower than 10 cfu/g) over time.

In addition, the stability of said compositions in the form of homogeneous solutions obtained by means of the method of the invention makes said compositions effective for the treatment of diseases and disorders of the respiratory airways which lead to overproduction of mucus, since there is no variation in the titre of the composition over time.

Being based on water and chitosan, the compositions of the invention are without side effects or contraindications, in particular for subjects in paediatric or adolescent age. Once applied by the user, the compositions of the present invention do not cause discomfort in the site or area of application, for example they do not cause discomfort in the mucous membrane of the nose or throat, such as the so-called irritation of the throat.

These and other objects which will be clear from the detailed description that follows, are attained by the method and the compositions of the present invention due to the technical characteristics claimed in the attached claims.

### BRIEF DESCRIPTION OF THE DRAWINGS.

Figure 1 describes the efficacy of interaction of a composition obtained according to the method of the invention with mucin at different time intervals and for different amounts of mucin.
Figures 2A to 2D show a comparison of the efficacy of the composition subject of the present invention with respect to a placebo composition, carried out by means of the clinical trial discussed in Example 3 below.
Figure 3 shows a comparison of the efficacy of the composition subject of the present invention with respect to a placebo composition, in relation to nasal itching, conducted through the clinical trial discussed in Example 3 below.

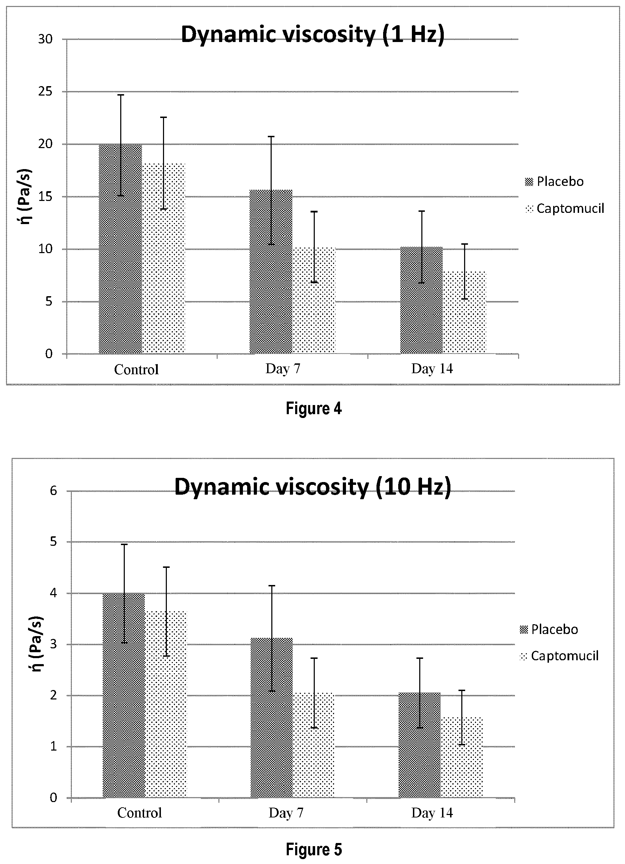

Figures 4 and 5 illustrate a comparison of the efficacy of the composition subject of the present invention with respect to a placebo composition, in relation to the dynamic viscosity of nasal mucus, conducted through the clinical trial discussed in Example 3 below.
In the context of the present invention, any numerical value referred to a physical or chemical quantity (for example pH, osmolality, molecular weight or degree of deacetylation) must be interpreted with a tolerance given by the measurement method used and known to the man skilled in the art. For example, a specific pH value should be considered with a tolerance of approximately ±1 or ±2 with respect to the specified pH value. For example, a specific molecular weight value is always referred to an average value of the preponderant fraction, the same applies in the case of the degree of deacetylation.

### DETAILED DESCRIPTION OF THE INVENTION

Forming an object of the present invention is a method for preparing a liquid composition in the form of a homogeneous solution comprising a chitosan comprising the steps of:
(I) preparing a solution (i) comprising water and a strong acid and having a pH value comprised in a range from 1 to 3, preferably from 1 to 2; followed by
(II) adding to said solution (i) - under stirring - a chitosan to obtain a solution (ii) comprising water and the chitosan and having an acid pH value comprised in a range from 1 to 4, preferably from 1.5 to 2.5; followed by
(III) adding a base to said solution (ii) until a homogeneous solution (iii) comprising water and chitosan and having a pH value comprised in the range from 5 to 6.8 (i.e. compatible with the human mucous membranes of the respiratory airways), preferably from 5.5 to 6.0; more preferably having a pH value of 5.8 ± 0.1.

Chitosan, which is normally a powder, is added to the strong acid aqueous solution of step (I). At the end of step (II) chitosan will be present as chitosan hydrochloride.
The liquid composition in the form of a homogeneous solution comprising a chitosan (composition of the invention), obtained by means of the method of the invention (steps (I)-(II)-(III) or (I)-(II)-(II.1)-(III)), comprises or, alternatively, consists of said homogeneous solution (iii).

According to an embodiment, the water used to prepare the solution (i) in step (I) is demineralised water. Preferably, the water used to prepare the solution (i) is mains water, subjected to a first demineralisation step and a subsequent second reverse osmosis step.

The method of the present invention does not comprise a step of adding an emulsifier. Consequently, the liquid composition of the invention is not an emulsion. The liquid composition that can be obtained by means of the method of the invention is a monophasic homogeneous solution (i.e. homogeneous solution (iii)). Preferably, said method does not comprise a step of adding citric acid, glycerophosphate, or a β-glycerophosphate sodium salt.

In chemistry, and in the present invention, a "solution" or "homogeneous solution" is defined as a homogeneous mixture in which one or more substances are contained in a liquid or solid or gaseous phase; a solution contains different particles mixed and evenly distributed in the space or volume available so that each volume of solution has the same composition as the other volumes of solution.

Therefore, in the context of the present invention, an administration of any volume of liquid composition ensures homogeneous and repeatable administration of such composition.

On the other hand, an "emulsion" is a dispersion of a fluid in the form of very small droplets in another non-miscible fluid (biphasic composition).

In an embodiment, besides the step (11.1) of adding an inorganic salt to said solution (ii), the method of the invention comprises, after step (II) and before carrying out step (III), preferably NaCl, up to bringing the osmolarity value of said solution (ii) to the range comprised from 250 mOsmoles to 950 mOsmoles, preferably from 350 mOsmoles to 850 mOsmoles, more preferably from 400 mOsmoles to 820 mOsmoles.

The osmolarity of the compositions of the invention was measured using standard methods known to the man skilled in the art, such as the following method: an osmolality is measured on 0.3 ml of composition, using an osmometer. Then, the measured osmolality is multiplied by the density of the composition, so as to obtain the osmolarity.
By way of example, an osmometer which can be used to measure osmolality in the aforementioned manner is an Emanuele Mires osmometer, model MIR300-P.

Advantageously, in order to obtain the desired osmolarity, in said step (11.1) the inorganic salt, preferably NaCl, is added at a percentage by weight comprised from 0.05% to 10% with respect to the total weight of the liquid composition of the invention, preferably comprised from 0.1% to 5%, more preferably comprised from 0.5% to 2%. The inorganic salt, for example NaCl, is added to stabilise the ionic strength of the solution. Only after which the pH value of the solution is raised using a strong base such as NaOH to a desired value, for example to pH 6.8 at most. Preferred examples of liquid compositions in the form of homogeneous solutions comprising a chitosan obtained by means of the method of the invention are compositions having osmolarity in the range from 400 to 640 mOsmoles, advantageously obtained by the addition of NaCl about 0.9% by weight, or from 550 to 820 mOsmoles, advantageously obtained by adding NaCl about 1.4% by weight (% by weight with respect to the total weight of the liquid composition).

Preferably, in said step (I) of the method of the invention the strong acid is hydrochloric acid (HCI), more preferably 1 M, 3 M or 5 M (M = molar).

Preferably, in said step (III) of the method of the invention, the base is a hydroxide of an alkaline metal or a hydroxide of an alkaline earth metal, preferably it is a sodium hydroxide (NaOH). According to an embodiment, a sodium hydroxide that can be used in this invention is in aqueous solution, preferably at 10% or at 30%.

The chitosan used in the method of the present invention can be of animal origin or of plant origin, preferably of plant origin, more preferably it is a fungal chitosan.

Said chitosan, preferably of plant origin (e.g. of fungal origin), has an average molecular weight comprised in the range from 50 KDa to 2000 KDa, preferably from 500 to 1500 KDa, more preferably from 800 KDa to 1200 KDa, such as, for example, 1000 KDa.

Said chitosan, preferably of plant origin (e.g. of fungal origin), has a degree of deacetylation comprised in the range from 50% to 100% by weight with respect to the weight of chitosan, preferably comprised from 70% to 100%, more preferably comprised from 90% to 100%, still more preferably comprised from 95% to 100% (e.g. 98%), for example it is a fungal chitosan deacetylated at 98% by weight.

The "degree of deacetylation" of chitosan means a percentage of basic lateral groups not blocked by the presence of the acetyl group; therefore, the higher the degree of deacetylation, the higher the percentage of free basic groups and, therefore, the higher the solubility of chitosan in acidic or slightly acidic aqueous solutions.

An example of a chitosan of plant origin that can be used in the present invention can be a chitosan with a degree of deacetylation ≥ 98%, with a viscosity comprised from 200 to 800 mpa.s, and with a pH value comprised from 7 to 8.

By way of example, a chitosan that can be used in the present invention could be the product "Chitosan", marketed by Willows Ingredients Ltd (Ireland).

In step (II) of the method of the invention said chitosan, preferably of plant origin (e.g. of fungal origin), is added at an amount such to be comprised in said liquid composition in the form of a homogeneous solution (or homogeneous solution (iii)) at a percentage by weight comprised in the range of from 0.01% to 10% with respect to the total weight of the liquid composition, preferably from 0.05% to 5%, more preferably from 0.1% to 1%, for example 0.16%.

In a preferred embodiment, the method of the invention comprises the steps of:
(I) preparing a solution (i) comprising water and HCL and having a pH value comprised in a range from 1 to 2; followed by
(II) adding to said solution (i) - under stirring - a chitosan deacetylated to 95-100% by weight (with respect to the weight of the chitosan) at an amount comprised from 0.1% to 1% by weight to obtain a solution (ii) comprising water and the chitosan and having an acid pH value comprised in a range from 1.5 to 2.5; keeping said solution (ii) under stirring for a time interval comprised from 30 minutes to 10 hours, preferably for 1 hour; followed by
(II.1) adding NaCl (0.5-2% by weight) to said solution (ii) until the osmolarity value of said solution (ii) is brought to the range comprised from 400 mOsmoles to 820 mOsmoles; followed by
(III) adding NaOH to said solution (ii) up to obtaining a homogeneous solution (iii) comprising water and the chitosan having a pH value at 5.8 ± 0.1 or 5.8 ± 0.2.
   unless otherwise specified, the percentages (%) by weight are with respect to the total weight of the liquid composition.

The method of the invention (steps (I)-(II)-(III) or steps (I)-(II)-(II.1)-(III)) further comprises a step (IV) of adding pharmaceutical or food grade additives and/or excipients, preferably after step (II) or step (11.1) and before step (IV). Preferably, said additives and/or excipients are selected from among flavours, sweeteners, preservatives (e.g. polylysines, paraben sodiums, nipagin sodium), cicatrising and mucosal protecting substances (e.g. dexpanthenol). Following the addition of additives and/or excipients, it is important that there be no increase in osmolarity of the composition of the invention, therefore, salts and all additives or formulation technology compounds which can dissociate and increase free charges in the liquid composition in the form of a homogeneous solution of the present invention should be avoided.

Forming an object of the present invention is a liquid composition in form of a homogeneous solution comprising a chitosan is a homogeneous solution comprising a chitosan, preferably of plant origin, that can be obtained by means of the method of the present invention (steps (I)-(II)-(III) or steps (I)-(II)-(II.1)-(III)), wherein said composition does not comprise an emulsifier.

The liquid composition in the form of a homogeneous solution of the invention comprises or, alternatively, consists of: a chitosan, preferably of plant origin, partially deacetylated (according to the percentages by weight reported in step (II) of the method), water, preferably an inorganic salt, for example NaCl, to adjust the osmolarity of the composition in a range comprised from 250 mOsmoles to 950 mOsmoles (preferably from 350 mOsmoles to 850 mOsmoles, more preferably from 400 mOsmoles to 820 mOsmoles) and, optionally, pharmaceutical or food grade additives and/or excipients. The liquid composition in the form of a homogeneous solution of the invention does not comprise an emulsifier.

The liquid composition in the form of a homogeneous solution of the invention may be a pharmaceutical composition or a medical device composition.

Forming an object of the present invention is the liquid composition in the form of a homogeneous solution not comprising an emulsifier, which can be obtained by means of the method of the present invention (steps (I)-(II)-(III) or steps (I)-(II)-(II.1)-(III)), for use in a method for the treatment of diseases and/or disorders of the respiratory airways, preferably selected from among the group comprising or, alternatively, consisting of illness, catarrh, common cold, flu, nasal infections, rhinitis, allergic rhinitis and sinusitis, through administration to a subject in need
In particular, the liquid composition in the form of a homogeneous solution comprising chitosan that can be obtained by means of the method of the invention (not comprising an emulsifier) achieves a high efficacy in the treatment of the aforementioned diseases and disorders related to cold in a short time (for example already after 30 seconds from the administration of the composition), since chitosan interacts with the mucin immediately after the composition is placed into contact with mucin samples, thus performing the fluidifying action, as reported in the experimental part.

A further object of the present invention is the liquid composition outlined in the Example 1, reported below, in particular in Examples 1A, 1B, 1C and 1D.

The liquid composition in the form of a homogeneous solution of the invention can be administered to said subject through the inhalation, oral or topical route; preferably through the inhalation route.

The liquid composition in the form of a homogeneous solution of the invention can be formulated as a nasal spray, oral spray, nasal drops, ear drops, oral solution, nasal wash solution, solution for ear washes, preferably it is a nasal spray.

The dosage of administration of the liquid composition in the form of a homogeneous solution of the invention to a subject will depend, for example, on: the condition to be treated, severity and course of the condition, previous therapy, age of the patient, clinical picture of the subject.

The liquid composition in the form of a homogeneous solution of the invention is administered to the subject at one or more times a day during the treatment period; it can be administered as a single treatment or in combination with other compositions or therapies (i.e. as coadjuvant) useful in the preventive and/or curative treatment of diseases and/or disorders of the respiratory airways.

Forming an object of the present invention is a liquid composition in the form of a homogeneous solution comprising a plant-based fungal chitosan and, optionally, pharmaceutical or food grade additives and/or excipients, having the characteristics as reported in the attached claims.

The plant-based chitosan present in said composition has at least (i) one degree of deacetylation comprised from 95% to 100% by weight, and (ii) one average molecular weight comprised from 1 KDa to 50 KDa. Said composition has at least one osmolality value comprised from 400 mOsmoles to 820 mOsmoles and a pH value comprised from 5.5 to 6.8. As the degree of deacetylation increases and as the average molecular weight of the plant-based fungal chitosan decreases, the more the composition obtained is clear, homogeneous without opalescence and stable over time.

In an embodiment said chitosan present in said composition has (i) a degree of deacetylation comprised from 98% to 100% by weight, and (ii) an average molecular weight comprised from 3 KDa to 30 KDa, preferably comprised from 5 KDa to 25 KDa, even more preferably comprised from 10 KDa to 20 KDa; and wherein said composition has an osmolality value comprised from 400 mOsmoles to 820 mOsmoles and a pH value comprised from 5.8 to 6.2.

The plant-based fungal chitosan, present in the composition of the present invention, has a chitosan fraction with an average molecular weight comprised from 3 kDa to 10 kDa, preferably comprised from 5 kDa to 10 kDa, greater than the chitosan fraction with an average molecular weight comprised from greater than 10 kDa to 25 kDa, preferably comprised from greater than 10 kDa to 20 kDa; furthermore, it has a weight ratio between the two fractions which can be comprised from 5:1 to 1:1, preferably from 4:1 to 1:1, or 3:1 to 1:1, or 2:1 to 1:1.
In an embodiment the liquid composition in the form of a homogeneous solution, according to the present invention, comprises (percentages (%) by weight with respect to the total weight of the composition):
- a plant-based fungal chitosan from 0.05% to 5%, preferably from 0.1% to 1%, for example: 0.16%, preferably with an average molecular weight comprised from about 3 kDa to about 30 kDa and a degree of acetylation comprised from about 95% to about 100%, preferably from about 98% to about 100%;
- 1 M hydrochloric acid comprised from 2.20% to 2.22% to prepare an aqueous solution with a pH value comprised from 1 to 2;
- apyrogenic sodium chloride comprise from 1.40% to 0.90% to prepare a solution with an osmolality value comprised from 400 mOsmoles to 820 mOsmoles;
- water: 92.90%-93.40%;
- additives and/or excipients: q.s. to 100%;
- NaOH: q.s. at pH from about 5.8 ± 0.1 to about 6.2 ± 0.1;
- a density of about 1.008 g/ml, at 25°C and P 1 Atm.

The liquid composition in the form of a homogeneous solution of the present invention comprises a preservative system capable of maintaining a low and stable microbial load of bacteria, moulds and yeasts each individually lower than 10 CFU/g, over time.

It is known that liquid compositions for spray use, for example for nasal sprays, are subjected to a step of irradiation with gamma rays step in order to sterilise the composition and maintain a low and stable microbial load of bacteria, moulds and yeasts each individually less than 10 CFU/g, over time.

However, the use of the gamma rays applied to the present composition causes the formation of an opalescence with the formation of a precipitate making the composition of the present invention inhomogeneous and opaque with all the resulting drawbacks.

The preservative system, contained in the composition of the present invention, allows instead to avoid subjecting the composition to a step of irradiation with gamma rays in order to sterilise said composition over time.

The preservative system can be represented, in an embodiment, by a mixture comprising or, alternatively, consisting of dexpanthenol (PubChem CID 131204; molecular formula C9H19NO4; synonyms Dexpanthenol, D-Panthenol 81-13-0, Pantothenol, D-Pantothenyl alcohol; molecular weight 205.25 g/mol) and nipagin sodium (sodium methylparaben for example E219; molar mass 174.13 g/mol, formula C8H7NaO3). However, other preservative systems can also be used in the context of the present invention if said systems allow to avoid subjecting the composition to gamma rays. Just like the preservative system described herein could contain other compounds, in addition to or in place of dexpanthenol and nipagin sodium, having similar functions and activities, provided that they allow to avoid subjecting the composition to gamma rays.

The composition of the present invention contains dexpanthenol and nipagin sodium at an amount by weight, respectively, comprised from 1% to 5% and from 0.1% to 0.3%; preferably comprised from 2% to 4% (for example 3%) and from 0.15% to 0.25% (for example 0.2%) by weight, with respect to the total weight of the composition.

Furthermore, besides the preservative system described above, the composition of the present invention comprises also the presence of a sweetening substance which allows to prevent said composition, once applied by the user, from causing discomfort in the site or area of application, for example, so as not to cause discomfort in the mucous membrane of the nose or throat or so-called irritation of the throat.

The sweetener selected and used in the composition of the present invention is saccharin (formula C7H5NO3S; molar mass 183.18 g/mol, IUPAC ID: 2H-1λ6,2-benzothiazol-1,1,3-trione, melting point 228.8°C, acidity (pKa): 1.6) in the form of sodium saccharin.

However, also other sweetening substances or compounds can be used in the context of the present invention if said sweetening substances or compounds allow to avoid causing discomfort to the mucous membrane at the site of application of the composition. Just like saccharin and/or sodium saccharin could be replaced, fully or partly, with another sweetening substance or compound, provided that the substitution allows to avoid causing discomfort to the mucous membrane of the nose or throat.

The composition of the present invention contains saccharin and/or sodium saccharin at an amount by weight comprised from 0.01% to 0.05% by weight, preferably comprised from 0.02% to 0.04%, (for example 0.03%) by weight, with respect to the total weight of the composition.

The composition of the present invention contains dexpanthenol and nipagin sodium at an amount by weight, respectively, comprised from 1% to 5% and from 0.1% to 0.3%; preferably comprised from 2% to 4% (for example 3%) and from 0.15% to 0.25% (for example 0.2%) and saccharin and/or sodium saccharin at an amount by weight comprised from 0.01% to 0.05% by weight, preferably comprised from 0.02% to 0.04% (for example 0.03%) by weight, with respect to the total weight of the composition.
In an embodiment the liquid composition in the form of a homogeneous solution, according to the present invention, comprises (percentages (%) by weight with respect to the total weight of the composition):
- a plant-based fungal chitosan from 0.05% to 5%, preferably from 0.1% to 1%, for example: 0.16%, preferably with an average molecular weight comprised from about 3 kDa to about 30 kDa and a degree of acetylation comprised from about 95% to about 100%, preferably from about 98% to about 100%;
- 1 M hydrochloric acid comprised from 2.20% to 2.22% to prepare an aqueous solution with a pH value comprised from 1 to 2;
- apyrogenic sodium chloride comprise from 1.40% to 0.90% to prepare a solution with an osmolality value comprised from 400 mOsmoles to 820 mOsmoles;
- water: 92.90%-93.40%;
- a preservative system comprising dexpanthenol and nipagin sodium at an amount by weight, respectively, comprised from 1% to 5% and from 0.1% to 0.3%; preferably comprised from 2% to 4% (for example 3%) and from 0.15% to 0.25% (for example 0.2%).
- a sweetening substance such as a saccharin and/or sodium saccharin at an amount by weight comprised from 0.01% to 0.05% by weight, preferably comprised from 0.02% to 0.04%, (for example 0.03%) by weight.
- additives and/or excipients: q.s. to 100%;
- NaOH: q.s. at pH from about 5.8 ± 0.1 to about 6.2 ± 0.1;
- a density of about 1.008 g/ml, at 25°C and P 1 Atm.

The liquid composition in the form of a homogeneous solution of the invention is administered to a subject (child, adolescent, adult or elderly) once or more times a day (for example 1 or 2 puffs for a total of about 0.13 ml per nostril 2 to 4 times a day; for example, for children, 1 puff for a total of about 0.07 ml per nostril 2 to 4 times a day) during the treatment period; it can be administered as a single treatment or in combination with other compositions or therapies (i.e. as coadjuvant) useful in the preventive and/or curative treatment of respiratory airways diseases and/or disorders.

The composition of the present invention is for use in the preventive and/or curative treatment of diseases and/or disorders of the respiratory airways, in particular in a method for treating diseases and/or disorders selected from among the group comprising or, alternatively, consisting of illness, catarrh, cold, flu, nasal infections, rhinitis, allergic rhinitis and sinusitis, through administration to a subject in need; preferably through administration by means of inhalation. E(n) preferred embodiments of the present invention are illustrated below:
E1. A liquid composition in the form of a homogeneous solution comprising a chitosan, wherein said chitosan is a plant-based fungal chitosan and it has:
   (i) a degree of deacetylation comprised from 95% to 100% by weight;
   (ii) an average molecular weight comprised from 1 KDa to 50 KDa; and wherein
      said composition has an osmolality value comprised from 400 mOsmoles to 820 mOsmoles and a pH value comprised from 5.5 to 6.8.
E2. The composition according to E1, wherein said chitosan has:
   (i) a degree of deacetylation comprised from 98% to 100% by weight;
   (ii) an average molecular weight comprised from 3 KDa to 30 KDa, preferably from 5 KDa to 25 KDa; and wherein said composition has an osmolality value comprised from 400 mOsmoles to 820 mOsmoles and a pH value comprised from 5.8 to 6.2.
E3. The composition according to E1 or E2, wherein said composition further comprises a preservative system represented by a mixture comprising, a or alternatively, consisting of dexpanthenol and nipagin sodium at an amount by weight, respectively, comprised from 1% to 5% and from 0.1% to 0.3%; preferably comprised from 2% to 4% (for example 3%) and from 0.15% to 0.25% (for example 0.2%) by weight, with respect to the total weight of the composition.
E4. The composition according to any one of the E1-E3, wherein said composition comprises a saccharin and/or sodium saccharin at an amount by weight comprised from 0.01% to 0.05% by weight, preferably comprised from 0.02% to 0.04%, (for example 0.03%) by weight, with respect to the total weight of the composition.
E5. A method for preparing a liquid composition in form of homogeneous solution comprising a chitosan comprising the steps of:
   (I) preparing a solution (i) comprising water and a strong acid and having a pH value comprised in a range between 1 and 3; followed by
   (II) adding to said solution (i) - under stirring - a plant-based fungal chitosan having a degree of deacetylation comprised from 95% to 100% by weight with respect to the weight of the chitosan, to obtain a solution (ii) comprising water and the chitosan and having an acid pH comprised in a range from 1 to 4; followed by
   (III) adding a base to said solution (ii) up to obtaining a homogeneous solution (iii) comprising water and the chitosan and having a pH value comprised in the range from 5.5 to 6.8.
E6. The method according to E5, wherein said method comprises the steps of:
   (I) preparing a solution (i) comprising water and HCL and having a pH value comprised in a range from 1 to 2; followed by
   (II) adding to said solution (i) - under stirring - a chitosan deacetylated to 98-100% by weight with respect to the weight of the chitosan, at an amount comprised from 0.1% to 1% by weight to obtain a solution (ii) comprising water and the chitosan and having an acid pH value comprised in a range from 1.5 to 2.5; keeping said solution (ii) under stirring for a time interval comprised from 30 minutes to 210 hours, preferably for 1 hour; followed by
   (II.1) adding NaCl to said solution (ii) at an amount by weight comprised from 0.5% to 2% by weight, until the osmolarity value of said solution (ii) is brought to the range comprised from 400 mOsmoles to 820 mOsmoles; followed by
   (III) adding NaOH to said solution (ii) up to obtaining a homogeneous solution (iii) comprising water and the chitosan and having a pH value comprised from 5.8 ± 0.1 to 6.2 ± 0.1.
E7. The method according to E5 or E6, wherein said method does not comprise a step of adding an emulsifier.
E8. The method according to any one of E5-E7, wherein said method does not comprise a step of adding citric acid, glycerophosphate, nor a β-glycerophosphate sodium salt.
E9. A liquid composition in the form of a homogeneous solution comprising a plant-based fungal chitosan that can be obtained by means of the method according to any one of E5-E8, wherein said composition does not comprise an emulsifier.
E10. The liquid composition in the form of a homogeneous solution according to any one of E1-E4 or E9 for use in a method for the treatment of diseases and/or disorders selected from among the group comprising or, alternatively, consisting of illness, catarrh, common cold, flu, nasal infections, rhinitis, allergic rhinitis and sinusitis, through administration to a subject in need; preferably through administration through inhalation route.

The present invention will now be illustrated based on the following examples, provided solely by way of non-limiting example.

### EXAMPLES.

### Example 1: Liquid composition according to an embodiment.

An example of an embodiment of a liquid composition in the form of a homogeneous solution, according to the present invention comprises (percentages (%) by weight with respect to the total weight of the composition):
- plant-based chitosan: 0.16%,
- 1M hydrochloric acid: 2.20%-2.22%
- apyrogenic sodium chloride: 1.40%-0.90%
- water: 92.90%-93.40%,
- additives and/or excipients: q.s. to 100%
- NaOH: q.s. at pH of about 5.8 ± 0.1;
- density: 1.008 g/ml.
Example of dosage of a liquid composition in the form of a homogeneous solution according to the Example reported above:
- for adults, 1 or 2 puffs for a total of about 0.13 ml per nostril 2 to 4 times a day.
- for children, 1 puff for a total of about 0.07 ml per nostril.

### Example 1A: Liquid composition (soft hypertonic) for nasal spray according to an embodiment.

An example of an embodiment of a liquid composition in the form of an aqueous homogeneous solution, according to the present invention comprises (percentages (%) by weight with respect to the total weight of the composition):
- plant-based fungal chitosan (% deacetylation at least 98% and molecular weight from 3 kDa to 30 kDa): 0.16%,
- 1M hydrochloric acid: 2.22%,
- apyrogenic sodium chloride: 0.90%,
- water: 92%-94%,
- additives and/or excipients: q.s. to 100%
- NaOH: q.s. at pH of about 5.8 ± 0.1
- density: 1.008 g/ml.
Net dose volume: 0.13 ml.
Net dose weight: 131 g.
Preparation method: add the plant-based chitosan under stirring to an aqueous solution of hydrochloric acid and wait for at least 1 hour; add sodium chloride and possible additives and/or excipients. Adjust the pH with NaOH slowly (NaOH at 30% after NaOH at 3% can be used up to pH 4.5).
Example of dosage of a liquid composition in the form of a homogeneous solution according to the Example reported above:
- for adults, 1 or 2 puffs for a total of about 0.13 ml per nostril 2 to 4 times a day.
- for children, 1 puff for a total of about 0.07 ml per nostril.

### Example 1B: Liquid composition (soft hypertonic) for nasal spray according to an embodiment.

An example of an embodiment of a liquid composition in the form of an aqueous homogeneous solution, according to the present invention comprises (percentages (%) by weight with respect to the total weight of the composition):
- plant-based fungal chitosan (% deacetylation at least 98% and molecular weight from 3 kDa to 30 kDa): 0.16%,
- 1M hydrochloric acid: 2.22%,
- sodium saccharin 0.03%,
- apyrogenic sodium chloride: 0.90%,
- dexpanthenol 3%,
- nipagin sodium (sodium methylparaben) 0.2%,
- water: 93%-94%,
- additives and/or excipients: q.s. to 100%
- NaOH: q.s. at pH of about 5.8 ± 0.1
- density: 1.008 g/ml.
Net dose volume: 0.13 ml.
Net dose weight: 131 g.
Preparation process: add the plant-based chitosan under stirring to an aqueous solution of hydrochloric acid and wait for at least 1 hour; add sodium chloride, nipagin sodium, dexpanthenol sodium saccharin and possible additives and/or excipients. Adjust the pH with NaOH slowly (NaOH at 30% after NaOH at 3% can be used up to pH 4.5).
Example of dosage of a liquid composition in the form of a homogeneous solution according to the Example reported above:
- for adults, 1 or 2 puffs for a total of about 0.13 ml per nostril 2 to 4 times a day.
- for children, 1 puff for a total of about 0.07 ml per nostril.

### Example 1C: Liquid composition (hypertonic) for nasal spray according to an embodiment.

An example of an embodiment of a liquid composition in the form of an aqueous homogeneous solution, according to the present invention comprises (percentages (%) by weight with respect to the total weight of the composition):
- plant-based fungal chitosan (% deacetylation at least 98% and molecular weight from 3 kDa to 30 kDa): 0.16%,
- 1M hydrochloric acid: 2.20%,
- apyrogenic sodium chloride: 1.40%,
- water: 92%-94%,
- additives and/or excipients: q.s. to 100%
- NaOH: q.s. at pH of about 5.8 ± 0.1
- density: 1.008 g/ml.
Net dose volume: 0.13 ml.
Net dose weight: 0.131 g.
Preparation process: add the plant-based chitosan under stirring to an aqueous solution of hydrochloric acid and wait for at least 1 hour; add sodium chloride and possible additives and/or excipients. Adjust the pH with NaOH slowly (NaOH at 30% after NaOH at 3% can be used up to pH 4.5).
Example of dosage of a liquid composition in the form of a homogeneous solution according to the Example reported above:
- for adults, 1 or 2 puffs for a total of about 0.13 ml per nostril 2 to 4 times a day.
- for children, 1 puff for a total of about 0.07 ml per nostril.

### Example 1D: Liquid composition (hypertonic) for nasal spray according to an embodiment.

An example of an embodiment of a liquid composition in the form of an aqueous homogeneous solution, according to the present invention comprises (percentages (%) by weight with respect to the total weight of the composition):
- plant-based fungal chitosan (% deacetylation at least 98% and molecular weight from 3 kDa to 30 kDa): 0.16%,
- 1M hydrochloric acid: 2.20%,
- sodium saccharin 0.03%,
- apyrogenic sodium chloride: 1.40%,
- dexpanthenol 3%,
- nipagin sodium (sodium methylparaben) 0.2%,
- water: 93%-94%,
- additives and/or excipients: q.s. to 100%
- NaOH: q.s. at pH of about 5.8 ± 0.1
- density: 1.008 g/ml.
Net dose volume: 0.13 ml.
Net dose weight: 0.131 g.
Preparation process: add the plant-based chitosan under stirring to an aqueous solution of hydrochloric acid and wait for at least 1 hour; add sodium chloride, nipagin sodium, dexpanthenol sodium saccharin and possible additives and/or excipients. Adjust the pH with NaOH slowly (NaOH at 30% after NaOH at 3% can be used up to pH 4.5).
Example of dosage of a liquid composition in the form of a homogeneous solution according to the Example reported above:
- for adults, 1 or 2 puffs for a total of about 0.13 ml per nostril 2 to 4 times a day.
- for children, 1 puff for a total of about 0.07 ml per nostril.

In the context of the present invention, the expression "subjects" is used to indicate human subjects or animal subjects (e.g. pets, such as dogs or cats or other mammals). Preferably, the compositions of the invention are for use in treatment methods for human subjects.

The expression "treatment method" in the context of the present invention is used to indicate an action, comprising the administration of a substance, or mixture of substances or combination thereof, with the aim of eliminating, reducing/decreasing or preventing a pathology or disease and its symptoms or disorders.

The expression "medical device" in the context of the present invention is used according to the meaning laid down by the Italian Legislative Decree n° 46, dated 24 February 1997 (or according to the new Medical Devices Regulation (UE) 2017/745 (MDR), i.e. it indicates a substance or another product, used alone or in combination, designated by the manufacturer to be used in humans for the diagnosis, prevention, control, therapy or attenuation of a disease, the product not exercising the main action, in or on the human body, for which it is designated, neither using pharmacological or immunology means nor by means of a metabolic process but the function thereof can be coadjuvated by such means.

The expression "component comprised in a range from x to y" is used to indicate that said component may be present, for example in the composition, at all the amounts present in said range, even if not stated, extremes of the range included.

### Example 2.

### EXPERIMENTAL PART

The Applicant carried out an analysis of the interactions between a liquid composition according to the invention based on chitosan in the form of a homogeneous solution according to Examples 1, 1A, 1B, 1C and 1D (Sample 1) and the soluble fraction of the pig gastric mucin. To this end, microviscosimetric calculations in diluted solution were used to evaluate the change in the relative viscosity (ηrel) at different time points as reported below.

### MATERIALS AND METHOD

- Mucin solution: pig gastric mucin (type III, bound sialic acid 0.5-1.5%, partially purified powder) and all standard grade chemical products are from Sigma-Aldrich (Munich, Germany). All the solutions were prepared in Ultrapure MilliQ water. The powdered mucin was hydrated in water through gentle magnetic stirring for 3 hours at room temperature and the insoluble fraction was removed by centrifugation at 25,000 × g for 50 minutes at 10°C.
The mucin concentration (∼ 26 mg/ml) was estimated to evaluate a relative viscosity (ηrel) of about 4.5.
- Sample 1 comprises: chitosan 0.16% (m/v) and NaCl 1.4% (m/v), degree of deacetylation 98%, density 1.008 g/ml (composition according to the invention).

### Method:

In order to evaluate the effectiveness of Sample 1 in terms of time, the variation in the relative viscosity (ηrel) of mixtures comprising Sample 1 in the presence of mucin at different time points was evaluated.
Sample 1 and mucin solution were mixed to form mixtures in different proportions, defined as the mass ratio f (f = [Mucin] / [Mucin] + [Chitosan]) in the range from f = 0 (i.e. only Sample 1) to f = 1.0 (i.e. mucin only). In detail, the mixtures of mass ratios f = 0.2, 0.4, 0.8 were evaluated, referring respectively to a weak, medium and severe cold, depending on the increasing amount of mucin in the mixture.
The mixtures (Sample 1 and mucin at f = 0.2, 0.4 and 0.8) were allowed to equilibrate at 37°C for 30 seconds while stirring at 400 rpm before starting the first viscosity measurements.
As controls, chitosan and mucin stock solutions were used and their ηrels were determined at t = 0 and at the longest time point.
The relative viscosity of the mixtures was measured as the average value of four analyses for each mixture at 37°C using an AMVn automatic rolling ball microviscometer (Anton Paar, Ostfildern, Germany) with programmable tube angle. The operation of this instrument is based on the principle of rolling ball time (that is, the time taken by a steel ball to roll through a fluid inside a calibrated capillary with a diameter of 1.8 mm held on a thermostated block). All viscosity measurements were recorded with a 50° tilt angle. The relative viscosity, ηrel, is calculated directly by measuring the lamination times of the polymeric solution (tₛₒₗᵤₜᵢₒₙ) and the solvent (tₛₒₗᵥₑₙₜ) (ηrel = tₛₒₗᵤₜᵢₒₙ / tₛₒₗᵥₑₙₜ).

### RESULTS

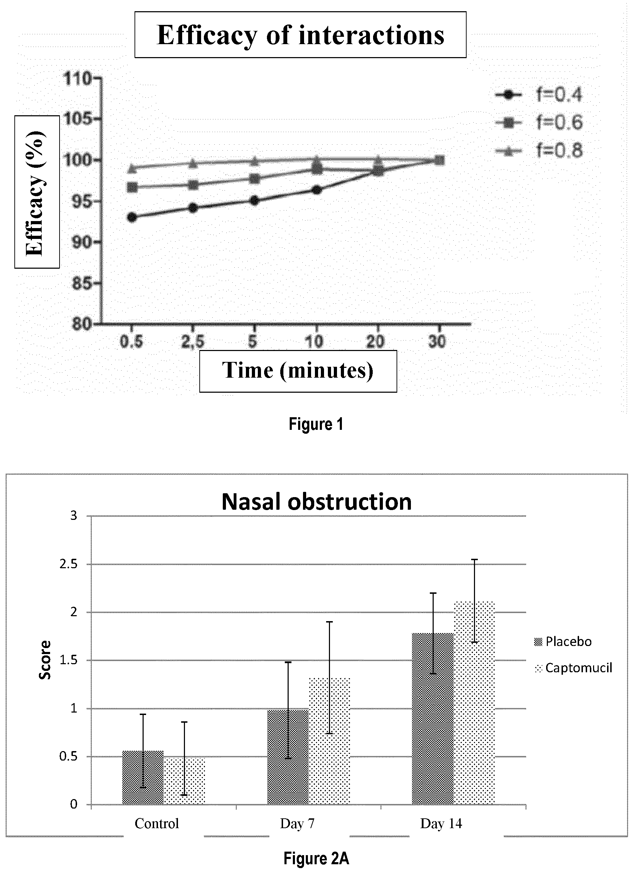

The results, reported in Figure 1, show that the liquid composition of the invention based on chitosan in the form of a homogeneous solution (Sample 1) interacts with the mucin immediately after being placed at contact, achieving an efficacy higher than 90% in 30 seconds (equivalent to a reduction in the viscosity and clearance of mucus in the nose).
This behaviour persists at all concentrations of mucin tested (f = 0.2, 0.4 and 0.8).
Since the magnitude and speed of the interaction were improved as mucin concentration increased, the results of this trial allow to conclude that the effect of the composition of the invention in the form of a homogeneous solution on the mucin increases in efficiency as the cold condition increases in severity (increased mucus secretion).

### Example 3: Clinical evaluation of the efficacy of the composition subject of the invention (Captomucil® nasal spray) according to Examples 1, 1A, 1B, 1C and 1D.

A randomised, double-blind trial was conducted in a sample of subjects with cold and rhinitis conditions.

The participants in the trial were selected by Cooperativa Medica Samnium (Benevento, Italy). The participants were aged between 18 and 75. The trial admission criteria were a total nasal symptom score (TNSS, defined hereinafter) ≤ 3, with a score for each symptom ≤ 2.

Exclusion criteria: structural nasal defects, hypertrophic nasal polyps (excluded via nasal endoscopy), pregnancy, breast-feeding and use of medicaments that would influence the results of the trial.

The trial lasted 12 weeks. The selection of subjects began in February 2019.

110 adult subjects (44 men and 66 women) were randomly divided into two groups with the same number of subjects (55): a GROUP A was administered the composition subject of the invention (Captomucil ®), a group B was administered a placebo composition.

The composition subject of the invention and the placebo composition were administered for a period of 14 days, with a frequency of one daily dose.

### Primary and secondary efficacy results.

The primary efficacy results measured involved changes in individual nasal symptoms (nasal obstruction, sneezes, and nose secretion), which were self-evaluated by the subjects.
For each symptom, the subjects recorded scores on daily cards according to the following four-level severity scale: 0 = severe; 1 = moderate; 2 = weak; 3 = absent.

The total nasal symptom score (TNSS) was calculated by adding the scores of the three individual nasal symptoms.

Nasal itching was evaluated separately, recording scores on daily self-evaluation cards according to the following six-level severity scale: 0 = absent; 1 = very low; 2 = low; 3 = moderate; 4 = high; 5 = severe.

Secondary efficacy results were collected by measuring mucus viscosity. In this regard, an oscillating sphere magnetic rheometer was used: each mucus sample to be analysed had a volume of 4 µl, and it was introduced into a test cell together with an iron microsphere having a radius of 100 µm. The dynamic viscosity (ή) of the mucus was calculated from the values of displacement amplitude of the microsphere and phase displacement with respect to the oscillatory driving force. Measurements were carried out at 25°C, near the ciliary beat frequency with (1 Hz) and without (10 Hz) mucus load.

The significance level (threshold value a) was 95% in all statistical analyses (P < 0.05).

### Results and discussion.

None of the 110 subjects ended their participation in the trial prematurely.

### Measure of the outcome of primary efficacy.

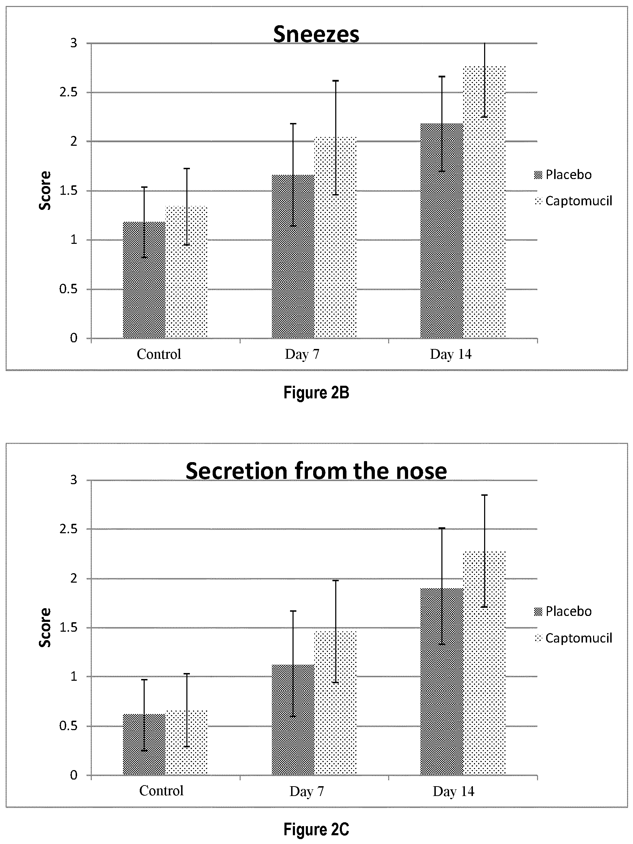

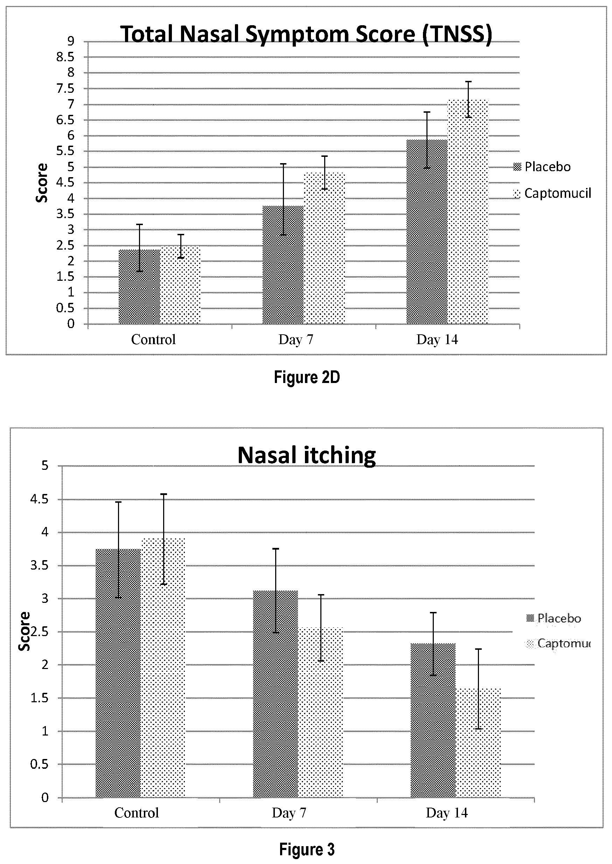

Figures 2A to 2D show how the composition subject of the present invention was able to improve the individual nasal symptoms of the subjects of GROUP A, with respect to the subjects of GROUP B, right from the first day of evaluation.
The trends at the middle and at the end of the observation period were as follows:
- Nasal obstruction: -34.7% (P < 0.005) (day 7) and -19.1% (P < 0.001) (day 14);
- Sneezes: -22.9% (P < 0.001) (day 7) and -26.6% (P < 0.001) (day 14);
- Secretion from the nose: -30.3% (P < 0.001) (day 7) and -20.0% (P < 0.001) (day 14);
- TNSS: -28.2% (P < 0.001) (day 7) and -22.2% (P < 0.001) (day 14).

Figure 3 shows a comparison of the efficacy of the composition subject of the present invention with respect to the placebo composition, in relation to nasal itching.
From this trial it is observed that the composition subject of the present invention was able to reduce nasal itching by 17.9% (P < 0.001) (day 7) and by 29.3% (P < 0.001) (day 14).

### Measure of the outcome of secondary efficacy.

The dynamic viscosity of the nasal mucus was determined using the oscillating sphere magnetic rheometer, at a frequency of 1 Hz and 10 Hz, so as to cyclically deform each mucus sample and observe the response of the mucus sample to the deformation.

The trends at the middle and at the end of the observation period were as follows:
- Dynamic viscosity at 1 Hz: -34.6% (P < 0.001) (day 7) and -22.7% (P < 0.001) (day 14);
- Dynamic viscosity at 10 Hz: -34.3% (P < 0.001) (day 7) and -23.4% (P < 0.001) (day 14).

From the previous trends it is therefore evident that the composition subject of the present invention selected from among those of Examples 1, 1A, 1B, 1C or 1D produced a primary efficacy and a secondary efficacy statistically relevant in the subjects belonging to GROUP A, with respect to the placebo composition administered to the subjects of GROUP B.

### Malvern Test

The compositions of Examples 1, 1A, 1B, 1C or 1D were subjected to the Malvern Test which allows to determine the droplet size. The Malvern Test is useful for demonstrating that the solution composition obtained by the method of the present invention (steps (I)-(II)-(III) or steps (I)-(II)-(II.1)-(III)) does not contain undissolved particles and, therefore, can be sprayed.
The following can be used: pump called PUMP_ASM-PAL/C-200/64-NN-AGD000001-A000-125-53,0--TE01 1,40-X--EMLJX5060A and nasal dispenser called ACT_ASM-CB20B-23,60--B31 BAG-3,90-19; or pump and dispenser manufactured by Silgan Dispensing SINFONIA.AG20.130.OR.PV24.GU. Ø19,5 TRISEAL F217-5 S0,75 and nasal dispenser called ERF20 *actuator.*
Particle size measured according to NR0614 Standard: "Malvern particle size" (TM-EU-Lab-012). Parameters:
- %<10 µm: percentage volume of spray with particles smaller than 10µm;
- Dv (10): particle size in which 10 % of the particles are smaller than this value;
- Dv (50): Mass Median Diameter (MMD);
- Dv (90): article size in which 90 % of the particles are smaller than this value;
- D [3][2]: Sauter mean diameter, ratio between total particle volume to total surface area;
- D [4][3]: mean diameter in µm derived from the volume distribution;
- Span: amplitude of the distribution of *the* particle size.
All the compositions tested successfully passed the test confirming the total absence of undissolved particles.

### Chitosan origin test.

The compositions subject of the present invention and, in particular, those of Examples 1, 1A, 1B, 1C or 1D, are obtained using fungal chitosan of plant origin. The origin was tested and confirmed. A sample of fungal chitosan of plant origin was subjected to analysis at 21°C to determine the presence of allergens using the ELISA technique (Crustaceans (E.L.I.S.A.) Method 07(S71) Rev. 13.2018). The result obtained is less than 0.05 mg/kg and it confirms the absence of chitosan of animal origin.

### Challenge Test.

The composition according to Example 1D (without 3% dexpanthenol, substituted with water) was subjected to a preliminary control (EU PHARMA 01/2011:50103 method to evaluate the efficacy of antimicrobial protection) to verify its suitability for a subsequent challenge test. Basically, it was found (see *Infra*) that the following challenge test could not be carried out on this sample because the sample was not microbiologically stable given that it had the following microbial count at T0:
i) MCIB01 - Total Mesophilic Bacteria Count cfu/g >3.0x104 UNI EN ISO 21149:2009
ii) MCIL01 - Yeast Count cfu/g <10 UNI EN ISO 16212:2011
iii) MCIM01 - Mould Count cfu/g <10 UNI EN ISO 16212:2011

As a matter of fact, the challenge test can only be carried out if the microbial count (bacteria, yeasts and moulds each individually) at T0 is < 10 CFU/gram.

The composition of Example 1D (with 3% dexpanthenol) has a microbial count (bacteria, yeasts and moulds each individually) at T0 < 10 CFU/gram. Therefore, the composition of Example 1D was subjected to the challenge test (EU Pharma 01/2011:50103).

The results of the challenge test carried out on the composition of Example 1D (hypertonic) are reported in Table 1 and Table 1a.

**Table 1**

| Microorganism | Control of Experimental Conditions | Control of the Toxicity of the Neutraliser | Validation of the dilution-neutralisation method |
|---|---|---|---|
| | A | B | C |
| *Staphylococcus aureus* ATCC 6538 | *x̅* : 74 | *x̅* : 72 | *x̅* : 77 |
| *Pseudomonas aeruginosa* ATCC 9027 | *x̅* : 49 | *x̅* : 50 | *x̅* : 46 |
| *Escherichia coli* ATCC 8739 | *x̅* : 63 | *x̅* : 64 | *x̅* : 61 |
| *Candida albicans* ATCC 10231 | *x̅* : 25 | *x̅* : 28 | *x̅* : 23 |
| *Aspergillus brasiliensis* ATCC 16404 | *x̅* : 54 | *x̅* : 54 | *x̅* : 49 |

**Table 1 a**

| COUNT FOR THE CLASSES OF MICROORGANISMS INOCULATED IN THE SAMPLE | | | | | | |
|---|---|---|---|---|---|---|
| | | microorganism | microorganism | microorganism | microorganism | microorganism |
| Step | | Gram+ *S.aureus* | Gram-*P.aeruginosa* | Gram-*E.coli* | Yeasts *C.albicans* | Moulds *A.brasiliensis* |
| 0 | CFU/g | 2.50E+06 | 4.60E+06 | 7.80E+06 | 3.60E+05 | 9.20E+05 |
| | Log | 6.40 | 6.66 | 6.89 | 5.56 | 5.96 |
| 2 | CFU/g | <10 | <10 | <10 | 9.00E+03 | 6.00E+04 |
| | Log | <1 | <1 | <1 | 3.95 | 4.78 |
| | *Reduction* | >5.4 | >5.66 | >5.89 | 1.60 | 1.19 |
| 7 | CFU/g | <10 | <10 | <10 | <10 | <10 |
| | Log | <1 | <1 | <1 | <1 | <1 |
| | *Reduction* | >5.4 | >5.66 | >5.89 | >4.56 | >4.96 |
| 14 | CFU/g | <10 | <10 | <10 | <10 | <10 |
| | Log | <1 | <1 | <1 | <1 | <1 |
| | *Reduction* | >5.4 | >5.66 | >5.89 | >4.56 | >4.96 |
| 28 | CFU/g | <10 | <10 | <10 | <10 | <10 |
| | Log | <1 | <1 | <1 | <1 | <1 |
| | *Reduction* | >5.4 | >5.66 | >5.89 | >4.56 | >4.96 |

The results of the challenge test carried out on the composition of Example 1B (soft hypertonic) are reported in Table 2 and Table 2a.

**Table 2**

| Microorganism | Control of Experimental Conditions | Control of the Toxicity of the Neutraliser | Validation of the dilution-neutralisation method |
|---|---|---|---|
| | A | B | C |
| *Staphylococcus aureus* ATCC 6538 | *x̅* : 74 | *x̅* : 72 | *x̅* : 73 |
| *Pseudomonas aeruginosa* ATCC 9027 | *x̅* : 49 | *x̅* : 50 | *x̅* : 48 |
| *Escherichia coli* ATCC 8739 | *x̅* : 63 | *x̅* : 64 | *x̅* : 61 |
| *Candida albicans* ATCC 10231 | *x̅* : 25 | *x̅* : 28 | *x̅* : 24 |
| *Aspergillus brasiliensis* ATCC 16404 | *x̅* : 54 | *x̅* : 54 | *x̅* : 56 |

**Table 2a**

| COUNT FOR THE CLASSES OF MICROORGANISMS INOCULATED IN THE SAMPLE | | | | | | |
|---|---|---|---|---|---|---|
| | | microorganism | microorganism | microorganism | microorganism | microorganism |
| Step | | Gram+ *S.aureus* | Gram-*P.aeruginosa* | Gram-*E.coli* | Yeasts *C.albicans* | Moulds *A.brasiliensis* |
| 0 | CFU/g | 2.50E+06 | 4.60E+06 | 7.80E+06 | 3.60E+05 | 9.20E+05 |
| | Log | 6.40 | 6.66 | 6.89 | 5.56 | 5.96 |
| 2 | CFU/g | <10 | <10 | <10 | 6.00E+03 | 9.00E+04 |
| | Log | <1 | <1 | <1 | 3.78 | 4.95 |
| | *Reduction* | >5.4 | >5.66 | >5.89 | 1.78 | 1.01 |
| 7 | CFU/g | <10 | <10 | <10 | <10 | 1.00E+03 |
| | Log | <1 | <1 | <1 | <1 | 3.00 |
| | *Reduction* | >5.4 | >5.66 | >5.89 | >4.56 | 2.96 |
| 14 | CFU/g | <10 | <10 | <10 | <10 | 9.00E+02 |
| | Log | <1 | <1 | <1 | <1 | 2.95 |
| | *Reduction* | >5.4 | >5.66 | >5.89 | >4.56 | 3.01 |
| 28 | CFU/g | <10 | <10 | <10 | <10 | <10 |
| | Log | <1 | <1 | <1 | <1 | <1 |
| | *Reduction* | >5.4 | >5.66 | >5.89 | >4.56 | >4.96 |

As can be seen from Tables 1a and 2a, at T14 (14 days after contamination T0) the microbial load is <10 CFU/g. This allows not to subject the composition of the invention to a step of irradiation with gamma rays.

A fungal chitosan solution of plant origin used in the composition of the present invention, for example in Example 1, was filtered on a 10,000 Dalton Molecular Weight Cut Off (MWCO) filter so as to obtain two CP solutions: a first solution containing chitosan having an average molecular weight higher than 10,000 Daltons (CP>10,000) and a second solution containing chitosan having an average molecular weight lower than 10,000 Daltons (CP<10,000). The two solutions were independently subjected to a GPC analysis. Analysis of the sample CP<10,000 Dalton and the sample CP>10,000 Dalton lead to the conclusion that the chitosan fraction having an average molecular weight distribution equal to or less than 10,000 Dalton, for example from 3 kDa to 10 kDa, is greater than the chitosan fraction having an average molecular weight comprised from greater than 10,000 Daltons to less than 25,000 Daltons. As the degree of deacetylation increases from 95% to 100% and as the average molecular weight of the plant-based fungal chitosan decreases from 3 kDA to 10kDa, the more the composition obtained is clear, homogeneous without opalescence and stable over time.

### EVALUATION OF THE MOLECULAR WEIGHT OF FUNGAL CHITOSAN (GPC-RID)

### Instruments:

Shimadzu chromatography unit, with pump, autosampler, refractive index detector and Lab solution data acquisition/processing system (or equivalent).
Column: PL aquagel-OH 60 8 µm 300 × 7.5 mm (or equivalent).

### Reagents:

Dextran according to GPC standard (AW ≈ 670000 Da or 25000);
Fungal chitosan, raw material;
Glacial acetic acid (CH3COOH);
Demineralised water.

### Parameters:

Mobile phase: CH3COOH in water 1% v/v.
Elution: isocratic
Column temperature: 30°C
Flow: 0.5 mL/min.
Injection volume: 20 ml
Processing time: 70 minutes

### Preparation of the solution

White: mobile phase
Standard solution: place approximately 15 mg of standard dextran in a 25 ml ampoule; add 10 ml of mobile phase and shake for approximately 2 minutes until complete dissolution. Filter the solution using an RC 0.45 µm filter before injection.
Test solution: place approximately 1g of sample in a 50 ml ampoule; add 20 ml of mobile phase and then sonicate the solution for 10 minutes. The sample solution may produce foam, wait for it to dissolve before bringing the solution to volume. Bring the solution to volume with the same solvent. Filter the resulting solution using a 10000 MWCO microcentrifuge filter. The filtered solution contains molecules with molecular weight (MW) MW<10000 Da (A), while the upper residue contains molecules with molecular weight (MW) MW >10000 Da (B).

### Procedure

Inject standard solutions twice and each sample solution only once.

## Claims

1. A liquid composition in the form of a homogeneous solution comprising a chitosan, wherein said chitosan is a plant-based fungal chitosan and it has:
(i) a degree of deacetylation comprised from 95% to 100% by weight;
(ii) an average molecular weight comprised from 1 KDa to 50 KDa; and wherein
said composition has an osmolality value comprised from 400 mOsmoles to 820 mOsmoles and a pH value comprised from 5.5 to 6.8.

2. The composition according to claim 1, wherein said chitosan has:
(i) a degree of deacetylation comprised from 98% to 100% by weight;
(ii) an average molecular weight comprised from 3 KDa to 30 KDa, preferably from 5 KDa to 25 KDa; and wherein said composition has an osmolality value comprised from 400 mOsmoles to 820 mOsmoles and a pH value comprised from 5.8 to 6.2.

3. The composition according to claim 1 or 2, wherein said composition further comprises a preservative system represented by a mixture comprising, a or alternatively, consisting of dexpanthenol and nipagin sodium at an amount by weight, respectively, comprised from 1% to 5% and from 0.1% to 0.3%; preferably comprised from 2% to 4% (for example 3%) and from 0.15% to 0.25% (for example 0.2%) by weight, with respect to the total weight of the composition.

4. The composition according to any one of the preceding claims, wherein said composition comprises a saccharin and/or sodium saccharin at an amount by weight comprised from 0.01% to 0.05% by weight, preferably comprised from 0.02% to 0.04%, (for example 0.03%) by weight, with respect to the total weight of the composition.

5. A method for preparing a liquid composition in form of homogeneous solution comprising a chitosan comprising the steps of:
(I) preparing a solution (i) comprising water and a strong acid and having a pH value comprised in a range between 1 and 3; followed by
(II) adding to said solution (i) - under stirring - a plant-based fungal chitosan having a degree of deacetylation comprised from 95% to 100% by weight with respect to the weight of the chitosan, to obtain a solution (ii) comprising water and the chitosan and having an acid pH comprised in a range from 1 to 4; followed by
(III) adding a base to said solution (ii) up to obtaining a homogeneous solution (iii) comprising water and the chitosan and having a pH value comprised in the range from 5.5 to 6.8.

6. The method according to claim 5, wherein said method comprises the steps of:
(I) preparing a solution (i) comprising water and HCL and having a pH value comprised in a range from 1 to 2; followed by
(II) adding to said solution (i) - under stirring - a chitosan deacetylated to 98-100% by weight with respect to the weight of the chitosan, at an amount comprised from 0.1% to 1% by weight to obtain a solution (ii) comprising water and the chitosan and having an acid pH value comprised in a range from 1.5 to 2.5; keeping said solution (ii) under stirring for a time interval comprised from 30 minutes to 210 hours, preferably for 1 hour; followed by
(II.1) adding NaCl to said solution (ii) at an amount by weight comprised from 0.5% to 2% by weight, until the osmolarity value of said solution (ii) is brought to the range comprised from 400 mOsmoles to 820 mOsmoles; followed by
(III) adding NaOH to said solution (ii) up to obtaining a homogeneous solution (iii) comprising water and the chitosan and having a pH value comprised from 5.8 ± 0.1 to 6.2 ± 0.1.

7. The method according to claim 5 or 6, wherein said method does not comprise a step of adding an emulsifier.

8. The method according to any one of claims 5-7, wherein said method does not comprise a step of adding citric acid, glycerophosphate, nor a β-glycerophosphate sodium salt.

9. A liquid composition in the form of a homogeneous solution comprising a plant-based fungal chitosan that can be obtained by means of the method according to any one of claims 5 to 8, wherein said composition does not comprise an emulsifier.

10. The liquid composition in the form of a homogeneous solution according to any one of claims 1-4 or 9 for use in a method for the treatment of diseases and/or disorders selected from among the group comprising or, alternatively, consisting of illness, catarrh, common cold, flu, nasal infections, rhinitis, allergic rhinitis and sinusitis, through administration to a subject in need; preferably through administration through inhalation route.
